# EUROPÄISCHE PATENTSCHRIFT

(11) **EP 3 263 551 B1**
(45) Veröffentlichungstag und Bekanntmachung des Hinweises auf die Patenterteilung: **14.10.2020**
(21) Anmeldenummer: 17175606.7
(22) Anmeldetag: 13.06.2017
(51) Int. Cl.: C07C 213/06, C07C 231/02, C07C 233/38, C07C 219/08

(54) **HERSTELLUNG VON N,N-(DI)ALKYLAMINOALKYL(METH)ACRYLAMID BZW. N,N-(DI)ALKYLAMINOALKYL(METH)ACRYLAT UND DEREN QUARTERNÄREN AMMONIUMSALZEN ALS FLOCKUNGSHILFSMITTEL UND GELBILDNER**
PREPARATION OF N, N- (DI) ALKYLAMINOALKYL (METH) ACRYLAMIDE OR N, N- (DI) ALKYLAMINOALKYL (METH) ACRYLATE AND THEIR QUATERNARY AMMONIUM SALTS AS FLOCCULANTS AND GELLING AGENTS
FABRICATION DE N,N-(DI)ALKYLAMINOALKYL(MÉTH)ACRYLAMIDE OU N,N-(DI)ALKYLAMINOALKYL(MÉTH)ACRYLAT ET LEURS SELS D'AMMONIUM QUATERNAIRE EN TANT QUE MOYEN AUXILIAIRES DE FLOCULATION ET GÉLIFIANTS

(30) Priorität: 28.06.2016 EP 16176555
(43) Veröffentlichungstag der Anmeldung: 03.01.2018
(73) Patentinhaber: Evonik Operations GmbH, 45128 Essen (DE)
(72) Erfinder: Krill, Steffen, 64367 Mühltal (DE); Hartmann, Patrik, 64572 Buettelborn (DE)
(74) Vertreter: Evonik Patent Association

(56) Entgegenhaltungen:
- EP-A1- 0 117 530
- EP-A2- 0 960 877
- WO-A1-03/101935
- WO-A2-99/25394
- DE-A1-102009 002 239
- US-A1- 2005 124 529
- US-A1- 2008 234 515
- ZHANG ANQIANG ET AL: "Synthesis and antimicrobial activities of acrylamide polymers containing quaternary ammonium salts on bacteria and phytopathogenic fungi", REACTIVE AND FUNCTIONAL POLYMERS, Bd. 88, 2015, Seiten 39-46, XP029214105, ISSN: 1381-5148, DOI: 10.1016/J.REACTFUNCTPOLYM.2015.02.005

## Beschreibung

Die vorliegende Erfindung beschreibt ein Verfahren zur Herstellung von N,N-(Di)alkylaminoalkyl(meth)acrylamid bzw. N,N-(Di)alkylaminoalkyl(meth)acrylat und deren quarternären Ammoniumsalzen mit geringem Gehalt der Verbindungen entsprechend der Formel (IV).

Die Herstellung von Dimethylaminopropylmethacrylamid (DMAPMA) ist aus dem Stand der Technik bekannt.
EP 0 960 877 (Elf Atochem S.A.) beschreibt ein kontinuierliches Verfahren zur Herstellung von Methacrylatestern von Dialkylaminoalkoholen. Man setzt Dialkylaminoalkohole mit im allgemeinen Methyl(meth)acrylat um und erhält das Dialkylaminoalkyl(meth)acrylat nach folgendem Verfahren:
Das Gemisch der Ausgangsstoffe (Methyl(meth)acrylat und Dialkylaminoalkohol) wird zusammen mit einem Tetraalkyltitanat als Katalysator (beispielsweise Tetrabutyl-, Tetraethyl- oder Tetra(2-Ethylhexyl)titanat) und mindestens einem Polymerisationsinhibitor (beispielsweise Phenothiazin, tert.-Butylcatechol, Hydrochinonmonomethylether oder Hydrochinon) kontinuierlich einem Rührreaktor zugeführt, wo bei einer Temperatur von 90°C - 120°C die Umsetzung zum Dialkylamino(meth)acrylat bei gleichzeitigem kontinuierlichem Abzug des azeotropen Methyl(meth)acrylat/Methanol-Gemisches erfolgt. Das rohe Reaktionsgemisch (Rohester) wird einer ersten Destillationskolonne zugeführt, wobei man bei vermindertem Druck am Kopf der Destillationskolonne einen im wesentlichen katalysatorfreien Strom abzieht und im Sumpf der Destillationskolonne der Katalysator sowie wenig Dialkylaminoalkyl(meth)acrylat abgezogen werden. Der Kopfstrom der ersten Destillationskolonne wird dann einer zweiten Destillationskolonne zugeführt, bei der man unter vermindertem Druck am Kopf einen Strom von niedrigsiedenden Produkten mit wenig Dialkylaminoalkyl(meth)acrylat und im Sumpf einen Strom bestehend aus hauptsächlich Dialkylaminoalkyl(meth)acrylat sowie Polymerisationsinhibitor(en) abzieht, der einer dritten Destillationskolonne zugeführt wird. In der dritten Destillationskolonne wird unter vermindertem Druck eine Rektifikation durchgeführt, in der man am Kopf den gewünschten reinen Dialkylaminoalkyl(meth)acrylatester und im Sumpf im wesentlichen den Polymerisationsinhibitor oder die Polymerisationsinhibitoren abzieht. Der Sumpfstrom der ersten Destillationskolonne wird nach weiterer Aufreinigung mit Hilfe eines Filmverdampfers genauso in den Reaktor zurückgeführt wie der Kopfstrom aus der zweiten Destillationskolonne.

Dieses Verfahren verzichtet auf eine Entwässerung der Alkohole vor dem Einsatz, was zu einer verstärkten Desaktivierung des eingesetzten Tetraalkyltitanats infolge Hydrolyse bis hin zur Bildung unerwünschter Feststoffniederschläge führen kann. Weiterhin verfügt das Verfahren über den Nachteil, dass in der ersten Destillationskolonne der Katalysator im Sumpf bei relativ hohen Temperaturen thermisch beansprucht wird. Dies kann leicht zur Zersetzung des Katalysators führen.
Bei diesem Verfahren werden sowohl die nicht umgesetzten Edukte wie auch das Produkt insgesamt zweimal über Kopf rektifiziert. Dies erfordert sehr hohe Energiekosten und insgesamt 4 Rektifikationskolonnen, die zum Teil sehr groß dimensioniert sein müssen. Der Prozeß ist daher mit sehr hohen Investitions- und Betriebskosten belastet.
EP 0 968 995 (Mitsubishi Gas Chemical Comp.) beschreibt ein kontinuierliches Verfahren zur Herstellung von Alkyl(meth)acrylsäureestern unter Verwendung einer Reaktionskolonne. Die Umesterungsreaktion erfolgt dabei direkt in einer Destillationskolonne (d.h. Reaktor und Destillationskolonne zum Abzug des Methyl(meth)acrylat/Methanol-Azeotrops bilden einen Apparat), der die Ausgangsstoffe (Methyl(meth)acrylat und Alkohol) kontinuierlich zugeführt werden. Der notwendige Katalysator, hier ebenfalls bevorzugt eine Titanverbindung, befindet sich in der Destillationskolonne. Im Fall eines homogenen Katalysators wird der Katalysator in die Destillationskolonne kontinuierlich eindosiert. Die Verwendung von homogenen Katalysatoren in einer Destillationskolonne führt jedoch aufgrund eines Spüleffektes durch den flüssigen Rücklauf in der Destillationskolonne zu einem erhöhten Katalysatorbedarf sowie bei Auftreten eines Katalysatorfeststoffniederschlags zur Verschmutzung der Kolonneneinbauten. Im Fall eines heterogenen Katalysators befindet sich der Katalysator in der Reaktionskolonne. Die Positionierung des Katalysators in der Destillationskolonne ist allerdings nachteilig, weil in der Destillationskolonne dann ein erhöhter Druckverlust auftritt und zusätzlich für die regelmäßige Reinigung der Destillationskolonne ein sehr hoher Aufwand betrieben werden muss. Weiterhin können heterogene Katalysatoren beispielsweise infolge unerwünschter Polymerisation deaktivieren.
In der US 8,674,133 (Evonik Röhm GmbH) wird ein kontinuierliches Verfahren zur Herstellung von Alkylamino(meth)acrylamiden mittels kontinuierlicher Aminolyse beschrieben. Die Verringerung des Vernetzergehaltes wird hierbei über aufwendige Aufbereitungsschritte, insbesondere Destillationen, erzielt.

Die vorstehend beschriebenen Verfahren führen zur Bildung verschiedener Nebenprodukte, die größtenteils nicht im Endprodukt verbleiben können. Die Abtrennung der Nebenprodukte führt zu den bekannten Nachteilen, wie beispielsweise Ausbeuteverluste, erhöhte Investitions-, Betriebs- und Wartungskosten durch die erforderlichen Aufreinigungsschritte.
Bei der Synthese von N,N-(Di)alkylaminopropylmethacrylamiden und deren quaternären Ammoniumsalzen ist die Bildung von Verbindungen der Formel (IV) 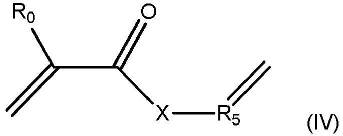 besonders unerwünscht. Ein erhöhter Anteil dieser Verbindung im Endprodukt führt bei der Polymerisation zur vorzeitigen und unkontrollierten Vernetzung.

Aufgabe war es, ein Verfahren zur Verfügung zu stellen, mit dem N,N-(Di)alkylaminoalkyl(meth)acrylamide bzw. N,N-(Di)alkylaminoalkyl(meth)acrylat und deren quaternären Ammoniumsalze mit geringem Gehalt an Verbindungen der Formel (IV) dargestellt werden können. Auch war es Aufgabe ein Verfahren zur Herstellung von Monomeren zur Verfügung zu stellen, welche die Herstellung von löslichen bzw. nicht koagulierenden Polymeren ermöglicht.

Gelöst wurde die Aufgabe durch ein Verfahren zur Herstellung von N,N-(Di)alkylaminoalkyl(meth)acrylamid bzw. N,N-(Di)alkylaminoalkyl(meth)acrylat der allgemeinen Formel (I) 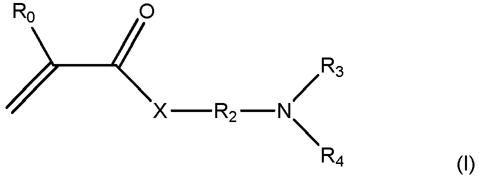 mit
R⁰ Wasserstoff oder Methyl
X NH oder O
R₂, R₃, R₄ jeweils ein linearer, verzweigter oder cyclischer Alkylrest, ein Arylrest, der auch mit ein oder mehreren Alkylgruppen substituiert sein kann; der lineare, cyclische oder verzweigte Alkylrest kann eine Länge von 1 - 12 Kohlenstoffatomen aufweisen, und beispielsweise Methyl, Ethyl, Propyl, Isopropyl, Butyl, Isobutyl, tert. Butyl, Pentyl, Hexyl, Heptyl, Octyl, Isooctyl, Nonyl, Decyl, Undecyl, Dodecyl,
mit einem Gehalt kleiner 1200 ppm der Verbindung (IV) der allgemeinen Formel 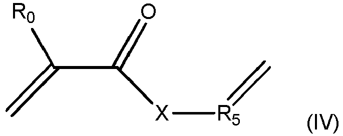 mit R₅ jeweils ein linearer, verzweigter oder cyclischer Alkylrest, ein Arylrest, der auch mit ein oder mehreren Alkylgruppen substituiert sein kann, der lineare, cyclische oder verzweigte Alkylrest kann eine Länge von 1 - 12 Kohlenstoffatomen aufweisen, und beispielsweise Methyl, Ethyl, Propyl, Isopropyl, Butyl, Isobutyl, tert. Butyl, Pentyl, Hexyl, Heptyl, Octyl, Isooctyl, Nonyl, Decyl, Undecyl, Dodecyl bedeuten;
wobei das Verfahren die die Aminolyse bzw. Alkoholyse von Alkyl(meth)acrylaten Formel (II) 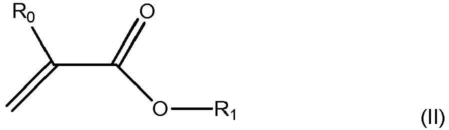 mit
R₀ Wasserstoff oder Methyl
R₁ linearem oder verzweigtem Alkylrest mit 1 bis 10, bevorzugt 1 bis 4 Kohlenstoffatomen
mit Aminen bzw. Alkoholen der Formel (III) 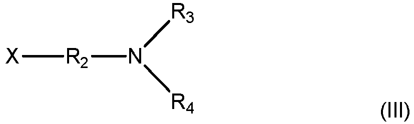 mit
X = OH oder NH₂; und
R₂, R₃, R₄ wie vorstehend beschrieben
unter Freisetzung von Alkoholen umfasst,
dadurch gekennzeichnet, dass die Sauerstoffkonzentration kleiner 1000 ppm in der Flüssigphase bei der Umsetzung der Edukte im Reaktionsbehälter oder der Rührkesselkaskade ist.

Gelöst wurde die Aufgabe durch ein Verfahren zur Herstellung von N,N-(Di)alkylaminoalkyl(meth)acrylamid bzw. N,N-(Di)alkylaminoalkyl(meth)acrylat der allgemeinen Formel (I) und deren quaternären Ammoniumsalzen mit einem Gehalt an Verbindungen der Formel (IV) kleiner 1200 ppm, dadurch gekennzeichnet, dass unter reduziertem Sauerstoffgehalt gearbeitet wird.

Bevorzugt ist der Gehalt der Verbindungen gemäß Formel (IV) kleiner 1000 ppm, besonders bevorzugt kleiner 850 ppm, ganz besonders bevorzugt kleiner 700 ppm.

Die Schreibweise (Meth)acrylat bedeutet hier sowohl Methacrylat, wie z.B. Methylmethacrylat, Ethylmethacrylat usw., als auch Acrylat, wie z.B. Methylacrylat, Ethylacrylat usw., sowie Mischungen aus beiden.

Gelöst wurde die Aufgabe auch durch ein Verfahren zur Herstellung von N, N-(Di)alkylaminoalkyl(meth)acrylamid oder N,N-(Di)alkylaminoalkyl(meth)acrylat gemäß Anspruch 1 mit einem Gehalt kleiner 1200 ppm der Verbindung der Formel (IV) oder deren quaternären Ammoniumsalze welche zur Herstellung von löslichen bzw. nicht koagulierenden Polymeren verwendet werden.

Lösliche bzw. nicht koagulierenden Polymere bedeutet in diesem Zusammenhang, dass die erfindungsgemäß hergestellten N,N-(Di)alkylaminoalkyl(meth)acrylamide bzw. N,N-(Di)alkylaminoalkyl(meth)acrylate nach der Polymerisation oder Copolymerisation mit anderen Verbindungen, in einem geeigneten Lösungsmittel in Lösung gebracht werden können bzw. eine nicht koagulierende Emulsion oder Dispersion bilden. Ebenso können die erfindungsgemäß quaternierten N,N-(Di)alkylaminoalkyl(meth)acrylamide bzw. N,N-(Di)alkylaminoalkyl(meth)acrylate nach Polymerisation oder Copolymerisation in geeigneten Lösungsmitteln in Lösung gebracht werden.

Überraschend wurde gefunden, dass die Bildung von Verbindungen der Formel (IV) durch die Reduzierung von Sauerstoff im Reaktionssystem minimiert werden kann.
Die Reduzierung von Sauerstoff bedeutet, dass bei der Herstellung von N,N-(Di)alkylaminoalkyl(meth)acrylamid bzw. N,N-(Di)alkylaminoalkyl(meth)acrylat in mindestens einem Teilschritt die Sauerstoffkonzentration <1000 ppm in der Flüssigphase ist. Bevorzugt ist die Sauerstoffkonzentration kleiner 1000 ppm in der Flüssigphase bei der Umsetzung der Edukte im Reaktionsbehälter oder der Rührkesselkaskade.
Idealerweise wird die Reaktion unter Ausschluß von Sauerstoff durchgeführt. Die Reaktion kann sowohl im Vacuum, als auch unter Schutzgasatmosphäre, beispielsweise unter N₂- oder ArgonAtmosphäre, durchgeführt werden.

Es wurde gefunden, dass auch die Verringerung der Verweilzeit zur Reduzierung der Bildung von Verbindungen der Formel (IV) beitragen kann. Eine Verringerung der Verweilzeit ohne erhebliche Verringerung der Raum-Zeit-Ausbeute kann durch folgende Maßnahmen erreicht werden:
- Einsatz aktiverer Katalysatoren,
- Einsatz erhöhter Katalysatormengen,
- eine Gleichgewichtsverschiebung durch verstärkten Alkohol-Abzug bspw. durch höhere Kolonnenkapazitäten und /oder mehr Energieeintrag.

Überraschend wurde gefunden, dass auch die Reduzierung der Reaktionstemperatur zur Verringerung des Gehalts an Verbindungen gemäß Formel (IV) führt. Um am Ende der Reaktion die verfahrensüblichen hohen Temperaturen zu vermeiden, wird gleichzeitig der Druck reduziert. Die Reduzierung des Drucks bei der Umsetzung erfolgt vorzugsweise gegen Ende der Reaktion. Dementsprechend kann der Druck zu Beginn der Reaktion auch erhöht werden.

Das Verfahren zur Herstellung von N,N-(Di)alkylaminoalkyl(meth)acrylamiden bzw. N,N-(Di)alkylaminoalkyl(meth)acrylat umfasst die Aminolyse bzw. Alkoholyse von Alkyl(meth)acrylaten mit Aminen bzw. Alkoholen unter Freisetzung von Alkoholen.

Der (Meth)acrylatfeed Edukt wird kontinuierlich einem geeigneten Reaktionsapparat zugeführt, wobei sowohl ein einzelner Reaktionsbehälter als auch eine Kaskade von mehreren hintereinander geschaltenen Reaktionsbehältern eingesetzt werden kann. Eine solche Kaskade kann beispielsweise aus 2, 3, 4, 5, 6 oder gegebenenfalls mehreren einzelnen Reaktionsbehältern bestehen. In einer bevorzugten Ausführungsform wird eine Kaskade aus 3 hintereinander angeordneten kontinuierlich betriebenen Rührkesseln verwendet.
Der (Meth)acrylatfeed Edukt kann auf verschiedene Weise erfolgen. Es ist zum Beispiel möglich, den Eduktstrom nur dem ersten Reaktionsbehälter der Kaskade zuzuführen oder aber den Eduktstrom in Teilströme aufzuteilen und diese Teilströme allen oder nur einigen der hintereinander geschalteten Reaktionsbehältern der Kaskade zuzuführen. Genauso ist es möglich, die Zuführung des Eduktstroms über die Leichtsieder-Austrags-Destillationskolonne und/oder die Reaktionsapparate vorzunehmen. Es kann vorteilhaft sein, den Eduktstrom nur in die Leichtsieder-Austrags-Destillationskolonne zuzuführen oder in einer weiteren Ausführungsform den Eduktstrom in Teilströme aufzuteilen, welche dann sowohl der Leichtsieder-Austrags-Destillationskolonne als auch dem ersten oder gegebenenfalls mehreren Reaktionsbehältern der Kaskade zugeführt werden.
Geeignete Alkyl(meth)acrylate sind Verbindungen der Formel (II) 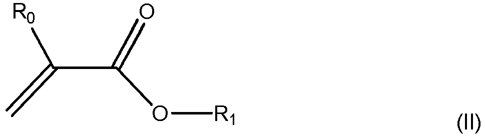 mit
- R₀: Wasserstoff oder Methyl
- R₁: linearem oder verzweigtem Alkylrest mit 1 bis 10, bevorzugt 1 bis 4 Kohlenstoffatomen

Typische Beispiele hierfür sind Methyl(meth)acrylat, Ethyl(meth)acrylat, Propyl(meth)acrylat, Isopropyl(meth)acrylat, n-Butyl(meth)acrylat, Isobutyl(meth)acrylat, 3-Methylbutyl(meth)acrylat, Amyl(meth)acrylat, Neopentyl(meth)acrylat, Hexyl(meth)acrylat, Cyclohexyl(meth)acrylat, Heptyl(meth)acrylat, n-Octyl(meth)acrylat, Ethylhexyl(meth)acrylat oder Decyl(meth)acrylat.

Es ist sinnvoll, dass alle Reaktionsbehälter einen Brüdenabzug zur Destillationskolonne zur Entfernung des bei der Reaktion frei werdenden Alkohols besitzen.
Es hat sich in besonderen Ausführungsformen als vorteilhaft erwiesen, den Austrag eines Kessels der Kaskade in den jeweils nachfolgenden Kessel der Kaskade von unten einzuführen.
Das Edukt wird kontinuierlich der Leichtsieder-Austrags-Destillationskolonne zur Entwässerung zugeführt.
Geeignete Edukte sind Verbindungen der Formel (III) 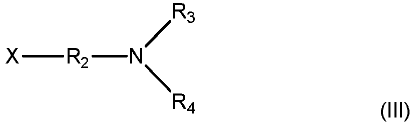 Mit
X = OH oder NH₂
R₂, R₃, R₄ jeweils ein linearer, verzweigter oder cyclischer Alkylrest, ein Arylrest, der auch mit ein oder mehreren Alkylgruppen substituiert sein kann, der lineare, cyclische oder verzweigte Alkylrest kann eine Länge von 1 - 12 Kohlenstoffatomen aufweisen, und beispielsweise Methyl, Ethyl, Propyl, Isopropyl, Butyl, Isobutyl, tert. Butyl, Pentyl, Hexyl, Heptyl, Octyl, Isooctyl, Nonyl, Decyl, Undecyl, Dodecyl sein.

Als Edukte kommen beispielsweise folgende Verbindungen in Frage:
Dimethylaminoethylamin, Diethylaminoethylamin, Dipropylaminoethylamin, Diisopropylaminoethylamin, Dibutylaminoethylamin, Disobutylaminoehtylamin, Dimethylaminopropylamin, Diethylaminopropylamin, Dipropylaminopropylamin, Diisopropylaminopropylamin, Dibutylaminopropylamin, Disobutylaminopropylamin, Dimethylaminobutylamin, Diethylaminobutylamin, Dipropylaminobutylamin, Diisopropylaminobutylamin, Dibutylaminobutylamin, Disobutylaminobutylamin, Dimethylaminohexylamin, Diethylaminohexylamin, Dimethylaminoneopentylamin, Methylethylaminopropylamin, Methylpropylaminopropylamin, Methylpropylaminoethylamin, Methylethylaminoethylamin. Dimethylaminoethylalkohol, Diethylaminoethylalkohol, Dipropylaminoethylalkohol, Diisopropylaminoethylalkohol, Dibutylaminoethylalkohol, Disobutylaminoehtylalkohol, Dimethylaminopropylalkohol, Diethylaminopropylalkohol, Dipropylaminopropylalkohol, Diisopropylaminopropylalkohol, Dibutylaminopropylalkohol, Disobutylaminopropylalkohol, Dimethylaminobutylalkohol, Diethylaminobutylalkohol, Dipropylaminobutylalkohol, Diisopropylaminobutylalkohol, Dibutylaminobutylalkohol, Disobutylaminobutylalkohol, Dimethylaminohexylalkohol, Diethylaminohexylalkohol, Dimethylaminoneopentylalkohol, Methylethylaminopropylalkohol, Methylpropylaminopropylalkohol, Methylpropylaminoethylalkohol, Methylethylaminoethylalkohol.

Besonders bevorzugt ist neben Dimethylaminopropylamin, Dimethylaminoethylamin, Dimethylaminohexylamin, Dimethylaminopropylalkohol, Dimethylaminoethylalkohol, und Dimethylaminohexylalkohol.

Katalysatoren und Polymerisationsinhibitoren werden ebenfalls bevorzugt kontinuierlich in den Reaktionsapparat zudosiert.
Als Umesterungskatalysatoren können alle aus dem Stand der Technik bekannten Katalysatoren eingesetzt werden.
Als Katalysatoren kommen beispielsweise Zirkonacetylacetonat und weitere 1,3-Diketone des Zirkons in Frage, ferner können Mischungen aus Alkalimetallcyanaten oder Alkalimetallthiocyanaten und Alkalimetallhalogeniden eingesetzt werden, ferner Zinnverbindungen, beispielsweise Dioctylzinnoxid, Erdalkalimetalloxide oder Erdalkalimetallhydroxide, wie beispielsweise LiOH, CaO, Ca(OH)₂, MgO, Mg(OH)₂ oder Gemische aus den vorstehend genannten Verbindungen, ferner Alkalimetallhydroxide, Alkalimetallalkoxide und Lithiumchlorid und Lithiumhydroxid, es können auch Gemische aus vorstehend genannten Verbindungen mit den vorstehend genannten Erdalkaliverbindungen und den Li-Salzen eingesetzt werden, insbesondere Lithiumchlorid und Calciumhydroxid, Dialkylzinnoxide, wie beispielsweise Dioctylzinnoxid, Alkalimetallcarbonate, Alkalimetallcarbonate zusammen mit quartären Ammoniumsalzen, wie beispielsweise Tetrabutylammoniumhydroxid oder Hexadecyltrimethylammoniumbromid, ferner Mischkatalysatoren aus Diorganylzinnoxid und Organylzinnhalogenid, saure Ionenaustauscher, Phosphormolybdän-Heteropolysäuren, Titanalkoholate, wie beispielsweise Isopropyltitanat, Chelatverbindungen der Metalle Titan, Zirkonium, Eisen oder Zink mit 1,3-DiCarbonylverbindungen, Bleiverbindungen, wie beispielsweise Bleioxide, Bleihydroxide, Bleialkoxide, Bleicarbonate oder Bleisalze von Carbonsäuren sowie Erdalkalimetallamide, insbesondere Lithiumamid. Ebenso sind Alkalimetallalkoholate, bevorzugt Lithiumalkoholate, insbesondere Lithiummethanolat geeignet. Besonders bevorzugt ist ein Katalysatorgemisch aus Dialkylzinnoxid und Alkyltitanat, beispielsweise Dioctylzinnoxid und Isopropyltitanat im Verhältnis ca. 1:3 (Gew.-%) bis 3:1 Gew.-%. Das Katalysatorgemisch wird in Mengen von 0,1-10 Gew.-%, bezogen auf die Menge der eingesetzten Edukte.
Als vorteilhaft hat sich eine Voraktivierung des Katalysators erwiesen. Dabei werden die Katalysatoren gemischt bzw. dispergiert, auf Temperaturen von 90°C bis 120°C erwärmt und für 2 bis 3 h gerührt, bis sich eine homogene, klare Lösung gebildet hat.

Als Polymerisationsinhibitoren kommen beispielsweise Hydrochinon, Hydrochinonmonomethylether und Piperidylderivate in Frage.
Die Reduzierung des Sauerstoffs erfordert gegebenenfalls, dass die üblicherweise verwendeten Stabilisatoren, die Sauerstoff benötigen, durch Stabilisatoren ersetzt werden müssen, die sauerstofffrei arbeiten.
Bevorzugt werden Polymerisationsinhibitoren ausgewählt aus der Gruppe Bis(2-methoxycarbonylpropyl)sulfid, N,N-Diethylhydroxylamin, Phenothiazin, 4-Hydroxy-2,2,6,6-tetramethylpiperidinooxyl oder N,N'-Diphenyl-p-phenylendiamin und deren Derivate (beispielsweise Ester, Amide usw.).

Die Wahl der Ausgangsstoffe erfolgt besonders vorteilhaft so, dass mit dem Entfernen des Alkohols aus der Reaktionsmischung das Gleichgewicht auf die Seite der Produkte verschoben werden kann. Die Entfernung des Alkohols kann destillativ durch seinen im Vergleich zum verwendeten Edukt niedrigeren Siedepunkt und/oder durch die Bildung eines Azeotrops erfolgen.

Das eingesetzte Edukt kann Wasser enthalten. Die Menge an Wasser im eingesetzten Edukt liegt unter 5000 ppm. Das Edukt wird vor dem Eintreten in den Reaktionsapparat bevorzugt über eine Destillationskolonne destillativ entwässert. Dabei wird das im Edukt enthaltene Wasser über Kopf abgezogen. Zur Vermeidung einer Verunreinigung des Niedersieder-Austrags mit dem eingesetzten Edukts erfolgt die Aufgabe des Edukts bevorzugt im unteren Teil der Destillationskolonne. Das eingesetzte Edukt kann auch auf andere Art und Weise entwässert werden:
- durch eine vorgeschaltete Entwässerungs-Destillationskolonne
   oder
- durch Behandlung mit einem Entwässerungsmittel, wie beispielsweise ein Molekularsieb,
   oder
- durch ein Membrantrennverfahren, wie beispielsweise eine Pervaporation.
Die Entwässerung ist daher bedeutsam, da das im Edukt enthaltene Wasser zur irreversiblen Schädigung des Katalysators im Reaktor führen kann. Das im Edukt enthaltene Wasser führt zur Bildung von Nebenprodukten und ist daher strikt zu vermeiden. Durch diesen Entwässerungsschritt vermeidet man die Hydrolyse des Katalysators und die damit entstehenden Kosten durch erhöhte Katalysatoreinsatzmengen und durch Probleme mit Feststoffniederschlägen. Außerdem wird die Reinheit des Produktes durch einen verringerten Anteil an Nebenprodukten erhöht.

Die Umsetzung erfolgt im Reaktionsapparat bei einer Temperatur im Bereich zwischen 70°C und 160°C je nach Stoffsystem und Betriebsdruck.

Erfindungsgemäß kann die Reaktionstemperatur verringert werden, um die Bildung von Verbindungen der Formel (IV) zu minimieren. Bevorzugt wird die Temperatur auf 80-120°C, besonders bevorzugt auf Temperaturen zwischen 80°C und 90°C verringert. Um einen möglichst vollständigen Umsatz zu erreichen, wird deshalb unter vermindertem Druck gearbeitet. Bei kontinuierlicher Verfahrensweise erfolgt die Umsetzung vorzugsweise in einer Kesselkaskade. Das Arbeiten unter reduziertem Druck kann in allen Kaskadenstufen erfolgen, vorzugsweise wird der Druck in der letzten Stufe der Kaskade reduziert.
Vorzugsweise wird im Vacuum zwischen 900 und 0 bar gearbeitet.

Erfindungsgemäß kann auch die Verringerung der Verweilzeit zur Reduzierung des Gehalts an Verbindungen gemäß Formel (IV) im Endprodukt führen.
Verweilzeiten zwischen 0,1 und 11 h, bevorzugt zwischen 1 und 5 h führen zur merklichen Reduzierung des Gehalts an Verbindungen gemäß Formel (IV) im Endprodukt.
Für einen ausreichend hohen Umsatz trotz verringerter Verweilzeit ist es erforderlich, dass aktivere Katalysatoren eingesetzt werden, oder/und die Katalysatormenge erhöht wird.
Zur Erhöhung der Reaktionsgeschwindigkeit wird der bei der Reaktion frei werdende Alkohol über eine Destillationskolonne aus dem Reaktionsgemisch, gegebenenfalls auch als Azeotrop mit dem Alkohol abgezogen. Dies kann sowohl bei atmosphärischem Druck oder bei Unterdruck erfolgen. Die Reaktionsmischung, welche größtenteils aus dem Produkt N,N-(Di)alkylaminoalkyl(meth)acrylamid bzw. N,N-(Dialkylaminoalkyl(meth)acrylat, nicht umgesetztem (Meth)acrylat und Edukt sowie geringen Mengen Alkohol, dem Katalysator, den Polymerisationsinhibitoren und einem Anteil an Nebenprodukten besteht, wird nach ca. 0,5 - 4 Stunden Reaktorverweilzeit, bevorzugt ist eine Verweilzeit von 1 - 2 Stunden, einem kontinuierlich betriebenen Fallfilmverdampfer zugeführt. Die Brüden des Fallfilmverdampfers werden einer Niedrigsieder-Destillationskolonne zugeführt. Dort erfolgt bei vermindertem Druck, bevorzugt im Bereich von ca. 1 mbar - 500 mbar, die Abtrennung der in Bezug auf das Produkt niedrigsiedenden Komponenten, vorwiegend Produktalkohol und nicht umgesetztes Edukt-(Meth)acrylat und Edukt Amin bzw. Alkohol. Diese werden über den Kopf der Destillationskolonne abgezogen und in den Reaktorbereich oder in die Destillationskolonne zurückgeführt. Durch diesen Kreislaufstrom wird ein hoher Umsatz bezogen auf die Edukte und den gesamten Prozess erzielt. Das im Ablauf des Fallfilmverdampfers anfallende mit Katalysator, Polymerisationsinhibitor und hochsiedenden Nebenprodukten noch verunreinigte Rohprodukt enthält vorzugsweise > 80 Gew.-% Produkt und wird zur Aufarbeitung einer weiteren Vakuumdestillationsstufe, welche im bevorzugten Druckbereich zwischen 0,1 und 200 mbar arbeitet, zugeführt. Hier erfolgt die destillative Abtrennung des reinen Produktes als Kopfprodukt. Als geeignete Apparate sind hierfür Fallfilm-, Dünnschicht- und Kurzwegverdampfer bekannt.

An die Herstellung der N,N-(Di)alkylaminoalkyl(meth)acrylamide bzw. N,N-(Di)alkylaminoalkyl(meth)acrylate kann sich gegebenenfalls noch eine Reindestillationsanlage anschließen, die auch unter vermindertem Druck, beispielsweise bei 500 - 0,1 mbar, betrieben werden kann. Dies ist insbesondere dann erforderlich, wenn eine besonders gute Abtrennung der im Prozess gebildeten, hochsiedenden Nebenkomponenten erfolgen soll.

Die erfindungsgemäß hergestellten N,N-(Di)alkylaminoalkyl(meth)acrylamid bzw. N,N-(Di)alkylaminoalkyl(meth)acrylat mit einem geringen Gehalt an Verbindungen der Formel (IV) können mit einem Alkylhalogenid oder Alkylsulfonat, vorzugsweise mit Methylchlorid, zum entsprechenden quaternären Ammoniumsalz umgesetzt werden.

N,N-(Di)alkylaminoalkyl(meth)acrylamid bzw. N,N-(Di)alkylaminoalkyl(meth)acrylat und deren quaternären Ammoniumsalze mit geringem Gehalt an Verbindungen der Formel (IV) eignen sich besonders für die Herstellung von Polymeren welche in Lösung gebracht werden oder aber in Lösung oder als Emulsion bzw. Dispersion polymerisiert werden.

Bevorzugt wird die Verwendung von N,N-(Di)alkylaminoalkyl(meth)acrylamid oder N,N-(Di)alkylaminoalkyl(meth)acrylat mit einem Gehalt der Verbindung gemäß Formel (IV) kleiner 1200 ppm hergestellt gemäß Anspruch 1 die zur Polymerisation als Bulkpolymerisation, Lösungspolymerisation, Dispersionspolymerisation bzw. Emulsionspolymerisation eingesetzt werden, und Anwendung als Verdicker (Thickener), Gelbildner (geling agent), Haarpflegemittel (Conditioning polymers), Fixative, Styling Polymer, Filmbildner (Film Former), Household / Industrial and Institutional Cleaning, Flockungsmittel (Flocculant), Klärmittel (Water Clarifier), Papierhilfsmittel ((paper auxiliaries /additives), Druckfarben, Fliesverbesserer in der Öl&Gas Industrie und als Gashydrat Inhibitoren, etc. finden.

Ebenfalls bevorzugt wird die Verwendung von N,N-(Di)alkylaminoalkyl(meth)acrylamid oder N,N-(Di)alkylaminoalkyl(meth)acrylat mit einem Gehalt der Verbindung gemäß Formel (IV) kleiner 1200 ppm hergestellt gemäß Anspruch 1 zur Umsetzung zu quaternären Ammoniumsalzen die zur Polymerisation eingesetzt werden, und Anwendung als Verdicker (Thickener), Gelbildner (geling agent), Haarpflegemittel (Conditioning polymers), Fixative, Styling Polymer, Filmbildner (Film Former), Household / Industrial and Institutional Cleaning, Flockungsmittel (Flocculant), Klärmittel (Water Clarifier), Papierhilfsmittel (paper auxiliaries /additives), Druckfarben, Fliesverbesserer in der Öl&Gas Industrie und als Gashydrat Inhibitoren, etc. finden.

Das erfindungsgemäße Verfahren wird durch die nachfolgenden Beispiele näher erläutert, ohne darauf beschränkt zu sein.

### Beispiele

### Beispiel 1

### Synthese von N,N-Dimethylaminopropylmethacrylamid (DMAPMA) unter Ausschluß von Sauerstoff

Zur kontinuierlichen Herstellung von N-Dimethylaminopropylmethacrylamid wird die Apparatur unter Stickstoff-Atmosphäre betrieben. Dem 1. Reaktionskessel werden 200 kg/h präaktivierter Katalysator-Feed mit einem Anteil von 2,0 Gew.-% Isopropyltitanat 5,0 Gew.-% Dioctylzinnoxid der Destillationskolonne und 144 kg/h N,N-Dimethylaminopropylamin (DMAPA) zudosiert. Die Präaktivierung wurde bei 110°C für 2h in einem Rührkessel durchgeführt. Weiterhin floss dem 1. Reaktionskessel über die Destillationskolonne der Kreislaufrückstrom vom Kopf der Niedrigsieder-Destillationskolonne kontinuierlich zu (400 kg/h mit der Zusammensetzung 70 Gew.-% Eduktmethacrylat sowie Methanol, DMAPA und Nebenprodukte. Das molare MMA: DMAPA-Verhältnis im Reaktorzulauf betrug 1,8 : 1. Weiterhin liefen die in der Destillationskolonne vom Methanol befreiten Brüden der Rührkessel über den Kolonnensumpf dem 1. Reaktionskessel zu. Unter diesen Reaktionsbedingungen (Druck ca. 500 mbar) stellte sich eine Reaktionstemperatur von 105°C im 1. Reaktionskessel ein. Die Reaktionstemperatur im 2. und 3. Reaktionskessel betrug 115 bzw. 125°C. Der Destillat-Abzug der Destillationskolonne betrug 110 kg/h.
Der Ablauf des 1. Reaktionskessels lief in den 2. Reaktionskessel und der Ablauf des 2. Reaktionskessels lief in den 3. Reaktionskessel. Die Brüden der einzelnen Reaktionskessel wurden kontinuierlich einer Destillationskolonne zugeführt.

Der Ablauf des 3. Reaktionskessels lief kontinuierlich dem Dünnschichtverdampfer einer Niedrigsieder-Kolonne zu, in der nicht umgesetztes DMAPA, Methylmethacrylat (MMA) und Methanol als Destillat (400 kg/h) abgezogen und als Kreislaufrückstrom der Destillationskolonne wieder zugeführt wurden. Der Sumpf-Auslauf des Dünnschichtverdampfers der Niedrigsiederkolonne betrug 230 kg/h und hatte die Zusammensetzung: 98,5 Gew.-% DMAPMA, < 1% Niedrigsieder, ca. 0,5% Hochsieder und 130 ppm N-Allylmethacrylamid.

### Beispiel 2

### Synthese von DMAPMA unter Ausschluß von Sauerstoff

Die Herstellung des Rohesters erfolgt entsprechend Beispiel 1.

Die Brüden der einzelnen Reaktionskessel wurden kontinuierlich einer Destillationskolonne zugeführt.

Der Ablauf des 3. Reaktionskessels lief kontinuierlich dem Dünnschichtverdampfer einer Niedrigsieder-Kolonne zu, in der nicht umgesetztes DMAPA, MMA und Methanol als Destillat (400 kg/h) abgezogen und als Kreislaufrückstrom der Destillationskolonne wieder zugeführt wurden. Der Sumpf-Auslauf des Dünnschichtverdampfers der Niedrigsiederkolonne betrug 230 kg/h und hatte die Zusammensetzung: 98,3 Gew.-% DMAPMA, < 1% Niedrigsieder, ca. 0,7% Hochsieder und 240 ppm N-Allylmethacrylamid.

### Vergleichsbeispiel 1

### Synthese von DMAPMA unter Sauerstoff

Zur kontinuierlichen Herstellung von N,N-Dimethylaminopropylmethacrylamid wurde dem 1. Reaktionskessel 200 kg/h präaktivierter Katalysator-Feed mit einem Anteil von 2,0 Gew.-% Isopropyltitanat 5,0 Gew.-% Dioctylzinnoxid der Destillationskolonne und 144 kg/h N,N-Dimethylaminopropylamin (DMAPA) zudosiert. Die Präaktivierung wurde bei 110°C für 2h in einem Rührkessel durchgeführt. Weiterhin floss dem 1. Reaktionskessel über die Destillationskolonne der Kreislaufrückstrom vom Kopf der Niedrigsieder-Destillationskolonne kontinuierlich zu (400 kg/h mit der Zusammensetzung 70 Gew.-% Eduktmethacrylat sowie Methanol, DMAPA und Nebenprodukte. Das molare MMA: DMAPA-Verhältnis im Reaktorzulauf betrug 1,8 : 1. Weiterhin liefen die in der Destillationskolonne vom Methanol befreiten Brüden der Rührkessel über den Kolonnensumpf dem 1. Reaktionskessel zu. Unter diesen Reaktionsbedingungen (Druck ca. 500 mbar) stellte sich eine Reaktionstemperatur von 138°C im 1. Reaktionskessel ein. Die Reaktionstemperatur im 2. und 3. Reaktionskessel betrug 143 bzw. 155°C. Der Destillat-Abzug der Destillationskolonne betrug 110 kg/h.

Der Ablauf des 1. Reaktionskessels lief in den 2. Reaktionskessel und der Ablauf des 2. Reaktionskessels lief in den 3. Reaktionskessel

Die Brüden der einzelnen Reaktionskessel wurden kontinuierlich einer Destillationskolonne zugeführt.

Der Ablauf des 3. Reaktionskessels lief kontinuierlich dem Dünnschichtverdampfer einer Niedrigsieder-Kolonne zu, in der nicht umgesetztes DMAPA, MMA und Methanol als Destillat (400 kg/h) abgezogen und als Kreislaufrückstrom der Destillationskolonne wieder zugeführt wurden. Der Sumpf-Auslauf des Dünnschichtverdampfers der Niedrigsiederkolonne betrug 240 kg/h und hatte die Zusammensetzung: ca. 90 Gew.-% DMAPMA, 0,1 Gew.-% DMAPA, 0,16 Gew.% N-Allylmethacrylamid sowie einen größeren Anteil hochsiedender Komponenten und Spuren der Reaktanten.

Das Rohprodukt wird anschließend in einer zweistufigen Destillation aufgearbeitet.

### Beispiel 3

### Laboruntersuchung des Einflusses von Sauerstoff auf die Bildung der Verbindung der Formel (IV)

DMAPMA wurde mit einer geeigneten Menge der genannten Stabilisatoren stabilisiert und jeweils ca. 10 g des stabilisierten DMAPMA in Reagenzgläser gefüllt. Jedes Reagenzglas enthält ein Einleitrohr zur Einleitung von Luft bzw. Stickstoff. Die Proben wurden nun in unterschiedlichen Ölbädern auf 110°C / 120°C / 130°C / 140°C und 150°C temperiert und über einen Zeitraum von 48 h Luft bzw. Stickstoff mit einem Gasstrom von jeweils ca. 0,75 L/h eingeleitet. Die Proben wurden abgekühlt und per GC untersucht. Der Gehalt an N-Allylmethacrylamid in DMAPMA ist in der nachfolgenden Tabelle in GC-Flächen-% dargestellt.

| Temperatur [°C] | Gehalt an N-Allylmethacrylamid in DMAPMA nach 48h / unter Luft [GC-FI.-%] | Gehalt an N-Allylmethacrylamid in DMAPMA nach 48h / unter Stickstoff [GC-FI.-%] |
|---|---|---|
| 110 | 1,69 | 0,34 |
| 120 | 7,87 | 0,33 |
| 130 | 11,08 | 0,32 |
| 140 | 21,97 | 0,32 |
| 150 | 23,54 | 0,33 |

### Beispiel 4

### Bestimmung der Grenzkonzentration

Herstellung eines Copolymeren aus DMAPMA und N-Allylmethacrylamid:
Das DMAPMA (enthaltend 130 ppm N-Allylmethacrylamid) wurde durch die Zugabe von N-Allylmethacrylamid auf eine Gesamtkonzentration von N-Allylmethacrylamid in der Mischung von 300ppm, 400ppm, 500ppm, 600ppm, 700ppm, 800ppm, 1000ppm, und 1200ppm aufkonzentriert. (N-Allylmethacrylamid, der Firma ABCR, Karlsruhe, Deutschland, Charge: 1300868)

Anschließend wurden jeweils 10g mit 0,2 % AIBN initiiert und bei 70°C in Reagenzgläsern im Wasserbad, innerhalb 3 h polymerisiert. Die Reagenzgläser wurden über Nacht auf Raumtemperatur abgekühlt und anschließend das Glas entfernt. Zur Lösung wurde ein Polymerstück entnommen.

Herstellung der wässrigen Lösungen:
Die jeweiligen Polymerstücke wurden in 125 mL Weithalsglasflaschen eingewogen und 3%ig bzw. 5%ig mit Wasser versetzt. Die Löslichkeit wurde nach 96h auf Vollständigkeit geprüft.

Die Viskosität der homogenen Lösungen wurden nach 96 h mittels Brookfield-Viskosimeter mit einem "small-sample" Adapter (Messvorrichtung für Lösungen mit niedriger Viskosität) gemessen.

| N-Allylmethacrylamid Gehalt | Viskosität [3%ig in H₂O] | Viskosität [5%ig in H₂O] |
|---|---|---|
| 130 ppm | 24 mPa*s | 35 mPa*s |
| 300 ppm | 28 mPa*s | 37 mPa*s |
| 400 ppm | 31 mPa*s | 45 mPa*s |
| 500 ppm | 33 mPa*s | 51 mPa*s |
| 600 ppm | 33 mPa*s | 60 mPa*s |
| 700 ppm | Nicht messbar* | Nicht messbar* |
| 800 ppm | Nicht messbar* | Nicht messbar* |

| | | |
|---|---|---|
| *Die Viskosität ist wegen der Unlöslichkeit der Polymere nicht messbar. | | |

## Patentansprüche

1. Verfahren zur Herstellung von N,N-(Di)alkylaminoalkyl(meth)acrylamid oder N,N-(Di)alkylaminoalkyl(meth)acrylat
der allgemeinen Formel (I) 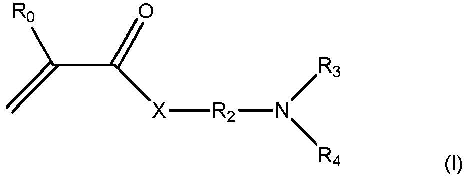 mit
R⁰ Wasserstoff oder Methyl
X NH oder O
R₂, R₃, R₄ jeweils ein linearer, verzweigter oder cyclischer Alkylrest, ein Arylrest, der auch mit ein oder mehreren Alkylgruppen substituiert sein kann, der lineare, cyclische oder verzweigte Alkylrest kann eine Länge von 1 - 12 Kohlenstoffatomen aufweisen, und beispielsweise Methyl, Ethyl, Propyl, Isopropyl, Butyl, Isobutyl, tert. Butyl, Pentyl, Hexyl, Heptyl, Octyl, Isooctyl, Nonyl, Decyl, Undecyl, Dodecyl bedeuten
mit einem Gehalt kleiner 1200 ppm der Verbindung (IV) der allgemeinen Formel 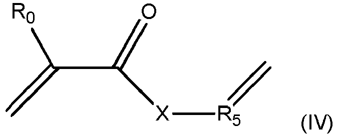 mit
R₅ jeweils ein linearer, verzweigter oder cyclischer Alkylrest, ein Arylrest, der auch mit ein oder mehreren Alkylgruppen substituiert sein kann, der lineare, cyclische oder verzweigte Alkylrest kann eine Länge von 1 - 12 Kohlenstoffatomen aufweisen, und beispielsweise Methyl, Ethyl, Propyl, Isopropyl, Butyl, Isobutyl, tert. Butyl, Pentyl, Hexyl, Heptyl, Octyl, Isooctyl, Nonyl, Decyl, Undecyl, Dodecyl bedeuten;
wobei das Verfahren die die Aminolyse bzw. Alkoholyse von Alkyl(meth)acrylaten Formel (II) 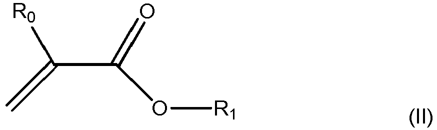 mit
R₀ Wasserstoff oder Methyl
R₁ linearem oder verzweigtem Alkylrest mit 1 bis 10, bevorzugt 1 bis 4 Kohlenstoffatomen
mit Aminen bzw. Alkoholen der Formel (III) 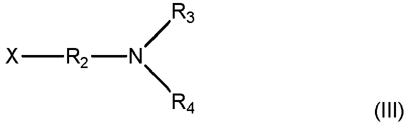 mit
X = OH oder NH₂; und
R₂, R₃, R₄ wie vorstehend beschrieben
unter Freisetzung von Alkoholen umfasst,**dadurch gekennzeichnet, dass** die Sauerstoffkonzentration kleiner 1000 ppm in der Flüssigphase bei der Umsetzung der Edukte im Reaktionsbehälter oder der Rührkesselkaskade ist.

2. Verfahren zur Herstellung von N,N-(Di)alkylaminoalkyl(meth)acrylamid oder N,N-(Di)alkylaminoalkyl(meth)acrylat gemäß Anspruch 1 mit einem Gehalt kleiner 1200 ppm der Verbindung der Formel (IV) oder deren quaternäre Ammoniumsalze welche zur Herstellung von löslichen bzw. nicht koagulierenden Polymeren verwendet werden.

3. Verfahren zur Herstellung von N,N-(Di)alkylaminoalkyl(meth)acrylamid oder N,N-(Di)alkylaminoalkyl(meth)acrylat gemäß Anspruch 2 mit einem Gehalt kleiner 1000 ppm.

4. Verfahren zur Herstellung von N,N-(Di)alkylaminoalkyl(meth)acrylamid oder N,N-(Di)alkylaminoalkyl(meth)acrylat gemäß Anspruch 1, **dadurch gekennzeichnet, dass** unter Ausschluß von Sauerstoff gearbeitet wird.

5. Verfahren zur Herstellung von N,N-(Di)alkylaminoalkyl(meth)acrylamid oder N,N-(Di)alkylaminoalkyl(meth)acrylat gemäß Anspruch 1, **dadurch gekennzeichnet, dass** die Reaktion unter Schutzgasatmosphäre durchgeführt wird.

6. Verfahren zur Herstellung von N,N-(Di)alkylaminoalkyl(meth)acrylamid oder N,N-(Di)alkylaminoalkyl(meth)acrylat gemäß Anspruch 1, **dadurch gekennzeichnet, dass** die Verweilzeit 0,1-11 h, bevorzugt 1-5 h beträgt.

7. Verfahren zur Herstellung von N,N-(Di)alkylaminoalkyl(meth)acrylamid oder N,N-(Di)alkylaminoalkyl(meth)acrylat gemäß Anspruch 1, **dadurch gekennzeichnet, dass** die Reaktion bei Temperaturen zwischen 70°C und 160°C durchgeführt wird.

8. Verfahren zur Herstellung von N,N-(Di)alkylaminoalkyl(meth)acrylamid oder N,N-(Di)alkylaminoalkyl(meth)acrylat gemäß Anspruch 6, **dadurch gekennzeichnet, dass** die Reaktion bei reduziertem Druck, bevorzugt im Vacuum durchgeführt wird.

9. Verfahren zur Herstellung von N,N-(Di)alkylaminoalkyl(meth)acrylamid oder N,N-(Di)alkylaminoalkyl(meth)acrylat gemäß Anspruch 1, **dadurch gekennzeichnet, dass** während der Umsetzung der Alkohol entfernt wird.

10. Verfahren zur Herstellung von N,N-(Di)alkylaminoalkyl(meth)acrylamid oder N,N-(Di)alkylaminoalkyl(meth)acrylat gemäß Anspruch 1, **dadurch gekennzeichnet, dass** Inhibitoren aus der Gruppe Bis(2-methoxycarbonylpropyl)sulfid, N,N-Diethylhydroxylamin, Phenothiazin, 4-Hydroxy-2,2,6,6-tetramethylpiperidinooxyl oder N,N'-Diphenyl-p-phenylendiamin oder deren Derivate eingesetzt werden.

## Claims

1. Process for preparing N,N-(di)alkylaminoalkyl(meth)acrylamide or N,N-(di)alkylaminoalkyl (meth)acrylate of the general formula (I) 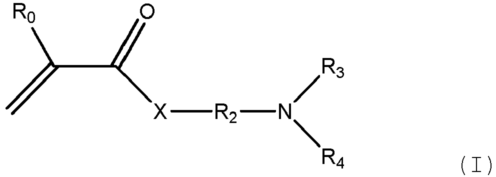 where
R⁰ is hydrogen or methyl
X is NH or O
R₂, R₃, R₄ are each a linear, branched or cyclic alkyl radical, an aryl radical which may also be substituted by one or more alkyl groups, the linear, cyclic or branched alkyl radical may have a length of 1-12 carbon atoms and is, for example, methyl, ethyl, propyl, isopropyl, butyl, isobutyl, tert-butyl, pentyl, hexyl, heptyl, octyl, isooctyl, nonyl, decyl, undecyl, dodecyl,
having a content of less than 1200 ppm of the compound (IV) of the general formula 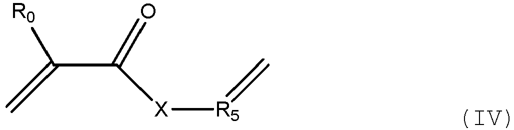 where R₅ in each case is a linear, branched or cyclic alkyl radical, an aryl radical which may also be substituted by one or more alkyl groups, the linear, cyclic or branched alkyl radical may have a length of 1-12 carbon atoms and is, for example, methyl, ethyl, propyl, isopropyl, butyl, isobutyl, tert-butyl, pentyl, hexyl, heptyl, octyl, isooctyl, nonyl, decyl, undecyl, dodecyl;
wherein the process comprises the aminolysis or alcoholysis of alkyl (meth)acrylates of the formula (II) 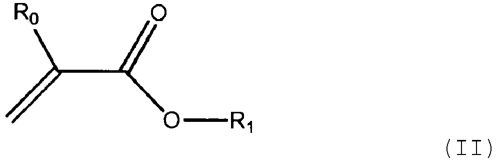 where
R₀ is hydrogen or methyl
R₁ is linear or branched alkyl radical having 1 to 10, preferably 1 to 4, carbon atoms
with amines or alcohols of the formula (III) 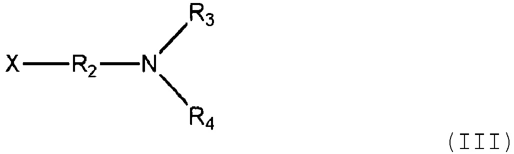 where
X = OH or NH₂; and
R₂, R₃, R₄ are as described above
with release of alcohols, **characterized in that** the oxygen concentration is less than 1000 ppm in the liquid phase in the conversion of the reactants in the reaction vessel or the stirred tank cascade.

2. Process for preparing N,N-(di)alkylaminoalkyl(meth)acrylamide or N,N-(di)alkylaminoalkyl (meth)acrylate according to Claim 1 having a content of less than 1200 ppm of the compound of the formula (IV) or quaternary ammonium salts thereof which are used for preparation of soluble or non-coagulating polymers.

3. Process for preparing N,N-(di)alkylaminoalkyl(meth)acrylamide or N,N-(di)alkylaminoalkyl (meth)acrylate according to Claim 2 having a content of less than 1000 ppm.

4. Process for preparing N,N-(di)alkylaminoalkyl(meth)acrylamide or N,N-(di)alkylaminoalkyl (meth)acrylate according to Claim 1, **characterized in that** operation is effected with exclusion of oxygen.

5. Process for preparing N,N-(di)alkylaminoalkyl(meth)acrylamide or N,N-(di)alkylaminoalkyl (meth)acrylate according to Claim 1, **characterized in that** the reaction is conducted under a protective gas atmosphere.

6. Process for preparing N,N-(di)alkylaminoalkyl(meth)acrylamide or N,N-(di)alkylaminoalkyl (meth)acrylate according to Claim 1, **characterized in that** the residence time is 0.1-11 h, preferably 1-5 h.

7. Process for preparing N,N-(di)alkylaminoalkyl(meth)acrylamide or N,N-(di)alkylaminoalkyl (meth)acrylate according to Claim 1, **characterized in that** the reaction is conducted at temperatures between 70°C and 160°C.

8. Process for preparing N,N-(di)alkylaminoalkyl(meth)acrylamide or N,N-(di)alkylaminoalkyl (meth)acrylate according to Claim 6, **characterized in that** the reaction is conducted under reduced pressure, preferably under vacuum.

9. Process for preparing N,N-(di)alkylaminoalkyl(meth)acrylamide or N,N-(di)alkylaminoalkyl (meth)acrylate according to Claim 1, **characterized in that** the alcohol is removed during the reaction.

10. Process for preparing N,N-(di)alkylaminoalkyl(meth)acrylamide or N,N-(di)alkylaminoalkyl (meth)acrylate according to Claim 1, **characterized in that** inhibitors from the group of bis (2-methoxycarbonylpropyl) sulphide, N,N-diethylhydroxylamine, phenothiazine, 4-hydroxy-2,2,6,6-tetramethylpiperidinooxyl or N,N'-diphenyl-p-phenylenediamine or derivatives thereof are used.

## Revendications

1. Procédé pour la préparation de N,N-(di)alkylaminoalkyl(méth)acrylamide ou de (méth)acrylate de N,N-(di)alkylaminoalkyle de formule générale (I) 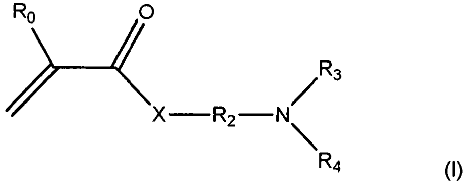 dans laquelle
R⁰ signifie hydrogène ou méthyle,
X signifie NH ou O,
R₂, R₃, R₄ signifient à chaque fois un radical alkyle linéaire, ramifié ou cyclique, un radical aryle, qui peut également être substitué par un ou plusieurs groupes alkyle, le radical alkyle linéaire, cyclique ou ramifié peut présenter une longueur de 1-12 atomes de carbone et par exemple signifier méthyle, éthyle, propyle, isopropyle, butyle, isobutyle, tert-butyle, pentyle, hexyle, heptyle, octyle, isooctyle, nonyle, décyle, undécyle, dodécyle,
présentant une teneur inférieure à 1200 ppm en composé de formule générale (IV) 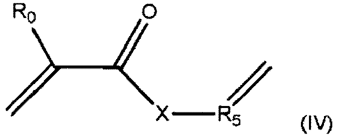 dans laquelle
R₅ signifie à chaque fois un radical alkyle linéaire, ramifié ou cyclique, un radical aryle, qui peut également être substitué par un ou plusieurs groupes alkyle, le radical alkyle linéaire, cyclique ou ramifié peut présenter une longueur de 1-12 atomes de carbone et par exemple signifier méthyle, éthyle, propyle, isopropyle, butyle, isobutyle, tert-butyle, pentyle, hexyle, heptyle, octyle, isooctyle, nonyle, décyle, undécyle, dodécyle ;
le procédé comprenant l'aminolyse ou, selon le cas, l'alcoolyse de (méth)acrylates d'alkyle de formule (II) 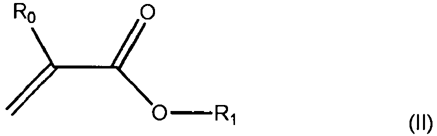 dans laquelle
R₀ signifie hydrogène ou méthyle,
R₁ signifie un radical alkyle linéaire ou ramifié comprenant 1 à 10, de préférence 1 à 4 atomes de carbone,
avec des amines ou, selon le cas, des alcools de formule (III) 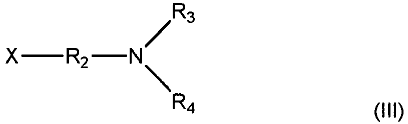 dans laquelle
X = OH ou NH₂ ; et
R₂, R₃, R₄ sont tels que décrits ci-dessus,
avec libération d'alcools, **caractérisé en ce que** la concentration en oxygène est inférieure à 1000 ppm dans la phase liquide lors de la transformation des produits de départ dans le récipient de réaction ou la cascade de cuves agitées.

2. Procédé pour la préparation de N,N-(di)alkylaminoalkyl(méth)acrylamide ou de (méth)acrylate de N,N-(di)alkylaminoalkyle selon la revendication 1, présentant une teneur inférieure à 1200 ppm du composé de formule (IV) ou de ses sels d'ammonium quaternaire qui sont utilisés pour la préparation de polymères solubles ou, selon le cas, non coagulants.

3. Procédé pour la préparation de N,N-(di)alkylaminoalkyl(méth)acrylamide ou de (méth)acrylate de N,N-(di)alkylaminoalkyle selon la revendication 2 présentant une teneur inférieure à 1000 ppm.

4. Procédé pour la préparation de N,N-(di)alkylaminoalkyl(méth)acrylamide ou de (méth)acrylate de N,N-(di)alkylaminoalkyle selon la revendication 1, **caractérisé en ce qu'**on travaille à l'abri d'oxygène.

5. Procédé pour la préparation de N,N-(di)alkylaminoalkyl(méth)acrylamide ou de (méth)acrylate de N,N-(di)alkylaminoalkyle selon la revendication 1, **caractérisé en ce que** la réaction est réalisée sous une atmosphère de gaz de protection.

6. Procédé pour la préparation de N,N-(di)alkylaminoalkyl(méth)acrylamide ou de (méth)acrylate de N,N-(di)alkylaminoalkyle selon la revendication 1, **caractérisé en ce que** le temps de séjour est de 0,1-11 h, de préférence de 1-5 h.

7. Procédé pour la préparation de N,N-(di)alkylaminoalkyl(méth)acrylamide ou de (méth)acrylate de N,N-(di)alkylaminoalkyle selon la revendication 1, **caractérisé en ce que** la réaction est réalisée à des températures entre 70 à 160°C.

8. Procédé pour la préparation de N,N-(di)alkylaminoalkyl(méth)acrylamide ou de (méth)acrylate de N,N-(di)alkylaminoalkyle selon la revendication 6, **caractérisé en ce que** la réaction est réalisée sous pression réduite, de préférence sous vide.

9. Procédé pour la préparation de N,N-(di)alkylaminoalkyl(méth)acrylamide ou de (méth)acrylate de N,N-(di)alkylaminoalkyle selon la revendication 1, **caractérisé en ce que** l'alcool est éliminé pendant la transformation.

10. Procédé pour la préparation de N,N-(di)alkylaminoalkyl(méth)acrylamide ou de (méth)acrylate de N,N-(di)alkylaminoalkyle selon la revendication 1, **caractérisé en ce que** des inhibiteurs du groupe sulfure de bis(2-méthoxycarbonylpropyle), N,N-diéthylhydroxylamine, phénothiazine, 4-hydroxy-2,2,6,6-tétraméthylpipéridinooxyle ou N,N'-diphényl-p-phénylènediamine ou leurs dérivés sont utilisés.
